# EUROPEAN PATENT APPLICATION

(11) **EP 4 122 467 A1**
(43) Date of publication of application: **25.01.2023**
(21) Application number: 21382669.6
(22) Date of filing: 22.07.2021
(51) Int. Cl.: A61K 31/522, A61K 45/06, A61P 35/00, A61P 35/04, G01N 33/50

(54) **THX-B FOR TREATING AND PREVENTING CANCER AND METASTASIS**

(71) Applicant: Fundación del Sector Público Estatal Centro Nacional de Investigaciones Oncológicas Carlos III (F.S.P. CNIO), 28029 Madrid (ES); Saragovi, Horacio Uri, Montréal, Québec H4A 3L8 (CA)
(72) Inventor: SARAGOVI, Horacio Uri, Montreal, Québec H4A3L8 (CA); PEINADO SELGAS, Héctor, 28029 Madrid (ES); GARCÍA SILVA, Susana, 28029 Madrid (ES); NOGUÉS VERA, Laura, 28029 Madrid (ES); HERNÁNDEZ BARRANCO, Alberto, 28029 Madrid (ES)
(74) Representative: Elzaburu S.L.P.

(57) **Abstract**

The present invention relates to a composition comprising THX-B and at least one pharmaceutically acceptable excipient for use in the treatment or prevention of cancer and/or metastasis.

## Description

### FIELD OF THE INVENTION

The present invention relates to a composition comprising THX-B and at least one pharmaceutically acceptable excipient for use in the treatment or prevention of cancer and/or metastasis.

### BACKGROUND

Metastasis is responsible of 90% of all cancer deaths. In metastatic melanoma, the 5-year survival is 15-20%. Although numerous studies have identified genes and mechanisms that control this process, the therapeutic options for this phase of the melanoma disease are limited. The FDA-approved treatments for metastatic melanoma include drugs inhibiting B-Raf/MAPK signalling, immunotherapy, treatment with Interleukin-2, Interferon alpha-derived compounds, DNA targeting agents and a viral oncolytic therapy. However, there are two types of limitations are relevant in melanoma therapy: a) adverse events, which can lead to skin and gastrointestinal toxicity, can be high and usually related to immune reactions and lack of specificity for tumor cells and b) reduced efficiency, which can occur due to resistance to immune, chemo/targeted and intralesional therapies. On top of that only specific subsets of patients can benefit from most common therapies. For example, only 40% patients respond to immunotherapy or B-Raf inhibitors are only prescribed for patients carrying the mutation.

The use of the compound known as THX-B (1,3-diisopropyl-1-[2-(1,3-dimethyl-2,6-dioxo-1,2,3,6-tetrahydro-purin-7-yl)-acetyl]-urea), which is a small molecule antagonist of the nerve Growth Factor Receptor (NGFR), helps to reduce melanoma metastasis.

US9296691 relates to the use of p75 receptor inhibitors where THX-B is described in the prevention of neurodegenerative disorders.

Vidal, A. et al., Cancers 2020, 12, 2460 (2020), relates to the use of a p75 inhibitor for the treatment or prevention of metastatic melanoma.

WO2011041453A1 relates to CD271 (p75 neurotrophin receptor) as a biomarker of melanoma cancer stem cells.

Zhong, M. et al, FEBS Open Bio, 2021 226―236 (2020), discloses that p75NTR-CTF may be a potential target protein for the treatment of patients with melanoma, and inhibitors of p75NTR proteolysis may favor the therapies.

AU2019203598A1 relates to the use of p75NTR inhibitors such as THX-B for treating inflammatory pain in arthritis, general inflammation and autoimmune diseases.

There is still a need for treatments improving the survival outcome of metastatic melanoma patients.

### DESCRIPTION OF THE INVENTION

The inventors have surprisingly found that the treatment with THX-B shows several differences compared with current melanoma therapies. Thus, instead of general chemotherapies or radiation therapy, it specifically targets NGFR. In addition, current melanoma treatment addressed against BRAF, MEK or PD1/ PD-L1 proteins reduce tumor growth. However, a wide number of melanoma patients acquire resistance to these therapies and develop more aggressive relapses that curses with the development of metastasis. Since NGFR is involved in invasion, metastasis and cancer stem cells, the technical effect of the present invention is that it blocks a completely different target that aims to inhibit metastatic spread of melanoma, which is still the main cause of death for this disease.

In a first aspect, the present invention relates to a composition comprising THX-B and at least one pharmaceutically acceptable excipient for use in the treatment or prevention of cancer and/or metastasis.

In a preferred embodiment of the first aspect, the composition comprising THX-B is administered intravenously, intraperitoneally, orally, subcutaneously, transdermally, subconjunctivally, topically or intravitreously.

In a preferred embodiment of the first aspect, the composition comprising THX-B is administered at a dose of between 0.1 and 100 mg/kg THX-B, preferably between 1 and 60 mg/kg THX-B, more preferably between 3 and 50 mg/kg THX-B. A preferred dose range when the composition is administered orally is from 25 to 100, more preferably from 40 to 60, even more preferably around 50mg/kg of THX-B. A preferred dose range when the composition is administered intraperitoneally is from 1 to 10, more preferably from 2 to 6, even more preferably around 4mg/kg of THX-B. The term "around" as used herein, means plus/minus 10 % of the indicated amount.

In a preferred embodiment of the first aspect, the cancer is a p75NTR expressing cancer. As used herein, the term "p75NTR" refers to p75 neurotrophin receptor (p75NTR), also known as CD271, a low-affinity receptor that, together with the tyrosine kinase receptor tropomyosin-receptor kinase (Trk), mediate neurotrophin (NT) functions. Preferably, the cancer is selected from pancreatic cancer, basal cell carcinoma, cervical squamous cell carcinoma (SCC), oral SCC, head and neck SCC, lung SCC, glioma, glioblastoma, esophageal SCC, medulloblastoma, breast cancer, thyroid cancer, ovarian cancer, renal cell carcinoma, malignant peripheral nerve tumors, schwannomas and melanoma or wherein the metastasis is from any one of pancreatic cancer, basal cell carcinoma, cervical squamous cell carcinoma (SCC), oral SCC, head and neck SCC, lung SCC, glioma, esophageal SCC, medulloblastoma, breast cancer, thyroid cancer, ovarian cancer, renal cell carcinoma, malignant peripheral nerve tumors, schwannomas and melanoma. More preferably, the cancer is melanoma or wherein the metastasis is from melanoma. In a preferred embodiment, the melanoma is skin melanoma.

In a preferred embodiment of the first aspect, THX-B is administered in combination with immunotherapy. Preferably, the immunotherapy is selected from inhibitors against CTLA-4, PD-1, PD-L1, Lag-3, Tim-3, TIGIT or a combination thereof, preferably inhibits CTLA-4, PD-1 or PD-L1, more preferably inhibits PD-L1.

As used herein, the term "CTLA-4" refers to a protein receptor found on T cells that functions as an immune checkpoint and downregulates immune responses. As used herein, the term "PD-1" refers to a protein is a protein on the surface of T and B cells that downregulates the immune system and promotes self-tolerance by suppressing T cell inflammatory activity. As used herein, the term "PD-L1" refers to is a cell membrane protein that binds to programmed cell death protein 1 (PD-1) on the effector T cells and transduces immunosuppressive signals. As used herein, the term "Lag-3" refers to is an immune checkpoint receptor protein found on the cell surface of effector T cells and regulatory T cells and functions to control T cell response, activation and growth. As used herein, the term "TIGIT" refers to an immune receptor present on some T cells and natural killer cells.

In a second aspect, the present invention relates to an early metastasis prognostic method comprising determining the expression levels of p75^{NTR} in a sample from a subject afflicted with cancer; wherein the sample comprises or consists essentially in tumor cells from the sentinel lymph node, circulating extracellular vesicles, circulating soluble p75^{NTR} or fragments thereof or circulating tumor cells; and wherein higher expression levels of p75^{NTR} in said sample are indicative of a worse prognosis.

In a preferred embodiment, the sample is a tumor cells sample from the sentinel lymph node or a tumor cells sample from circulating tumor cells and a count equal or above 75 tumor cells positive for p75^{NTR} is indicative of a worse prognosis, while a count below 75 tumor cells positive for p75^{NTR} is indicative if a better prognosis.

In a preferred embodiment, the expression levels of p75^{NTR} in the sample from a subject afflicted with cancer are compared with said expression levels in a subject not afflicted with cancer.

In a preferred embodiment, the sample can be a biopsy from a sentinel lymph node, a plasma sample, exudative seroma obtained from lymphatic drainage, urine, cerebrospinal fluid, vitreous humor or peritoneal lavage.

In a preferred embodiment of the second aspect, the cancer is melanoma. In another preferred embodiment, the subject is human.

In a preferred embodiment of the second aspect, the p75^{NTR} expression levels are analysed by immunohistochemistry, qPCR, flow cytometry or quantitative ELISA. As used herein, the term "qPCR" refers to refers to quantitative PCR which is a quantitative method, also known as Real-Time PCR, to detect, characterize and quantify DNA in real-time. As used herein, the term "immunohistochemistry" refers to a technique for detection and visualization of proteins, or other antigens, in tissue using antibodies specifically recognizing the target of interest.

In another aspect, the present invention relates to a composition comprising THX-B and at least one pharmaceutically acceptable excipient of the first aspect for use in the treatment or prevention of cancer and/or metastasis in a subject afflicted with cancer who has a worse prognosis in the early metastasis prognostic method of the second aspect.

In another aspect, the present invention relates to a composition comprising THX-B and at least one pharmaceutically acceptable excipient for use in the prevention of lymphangiogenesis. Preferably, the composition of the invention is used to reduce and/or prevent tumor lymphangiogenesis dependent on p75^{NTR}. In a preferred embodiment, it is used for the prevention of lymphatic disorders dependent on p75^{NTR} .

In another aspect, the present invention relates to a composition comprising THX-B and at least one pharmaceutically acceptable excipient for use in the treatment of diabetes and preferably for improving bladder function in diabetic patients.

### DESCRIPTION OF THE DRAWINGS

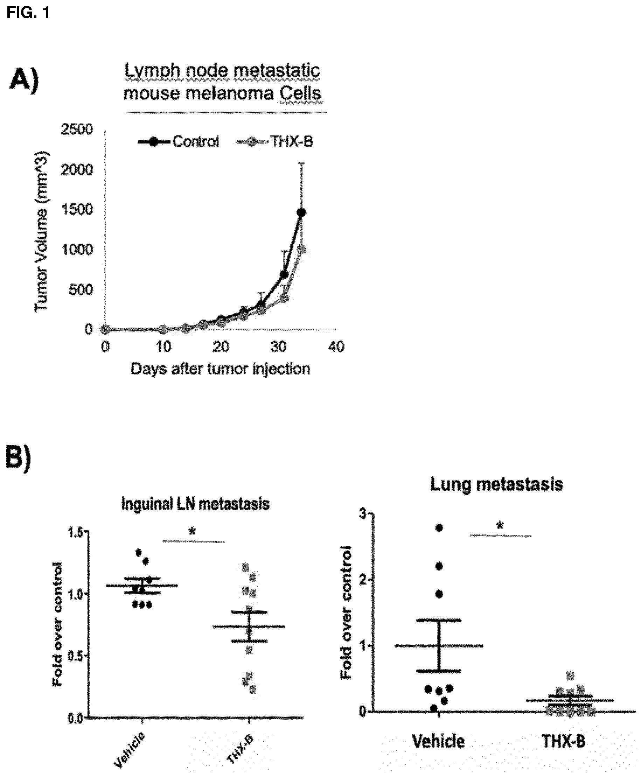

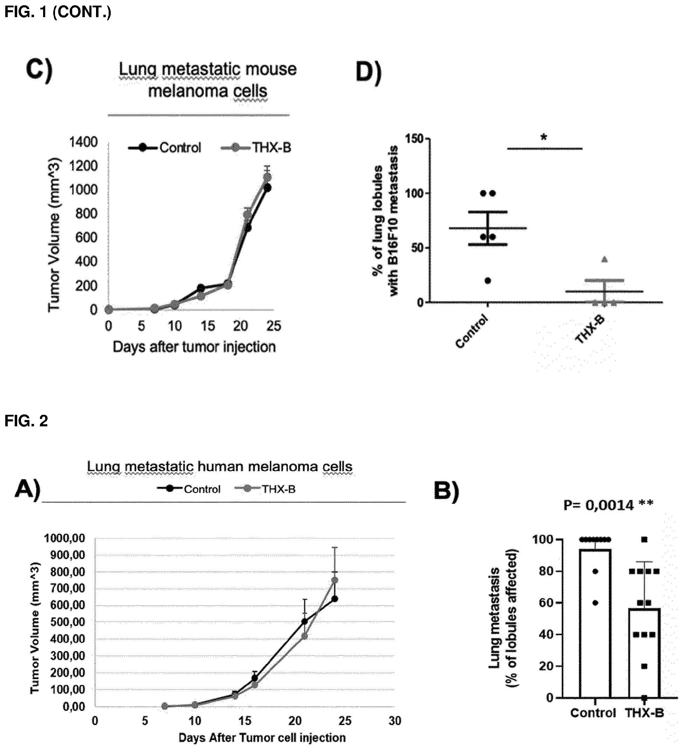

**Figure 1****. NGFR inhibition by THX-B reduces melanoma spontaneous metastasis.** A-B) 200.000 lymph node metastatic mouse melanoma cells were subcutaneously in c57/BL6 mice. Animals were treated intra-peritoneally with THX-B (4 mg/Kg twice a week), starting on day 7 post-injection of tumor cells. Tumor volume (A) was measured twice a week using a caliper. LN and lung metastatic burden (B) were measured using the IVIS image system and represented as fold over vehicle treated mice. Error bars represent s.e.m. of 2 experiments (n = 8 mice, vehicle group and n = 10, THX-B group). C-D) Similarly, tumor growth (C) was measured in mice subcutaneously injected with lung metastatic mouse melanoma cells (200.000) and treated with vehicle or THX-B in the same conditions as A) (n=4-5mice per condition). D) Percentage of lung lobules with melanoma metastasis in animals with flank tumors originated by injecting B16F10 cells. p values were calculated by two-tailed Student's t test.
**Figure 2****. NGFR inhibition by THX-B reduces melanoma spontaneous metastasis in immunodeficient models.** Lung metastatic human melanoma cells (500.000) were injected subcutaneously into the flank of nude mice. 7 days after s.c. injection, THX-B 4 mg/Kg or vehicle was intraperitoneal administrated twice a week. A) Tumor growth was measured with a caliper twice a week. B) Quantification of the percentage of lung lobules with melanoma metastasis was analyzed measuring Luc expression of tumor loci with the IVIS system. *p* values were calculated by two-tailed Student's t test.

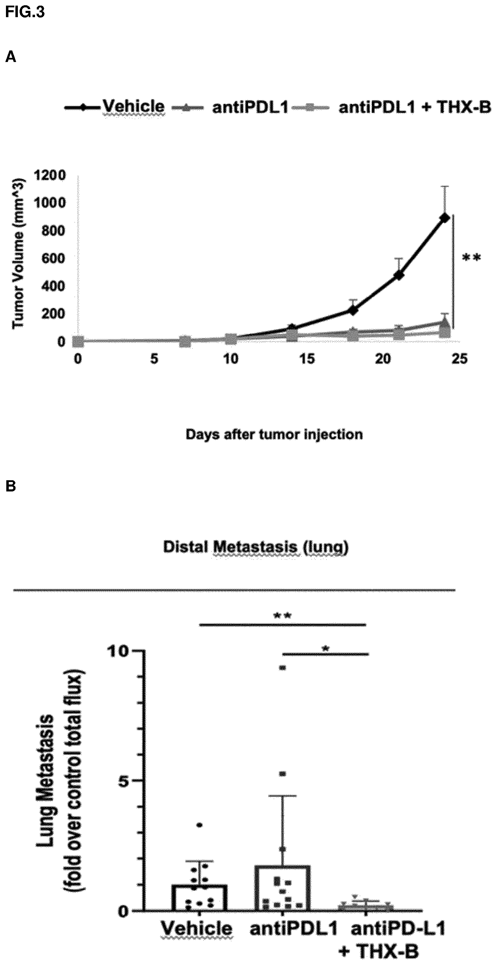

**Figure 3****. NGFR inhibition by THX-B in combination with immunotherapy (antiPDL1) reduces metastasis.** At day 7 after s.c mouse melanoma cells (200.000) injection in C57/BL6 mice, anti-PD-L1(5mg/Kg) alone or in combination with anti-NGFR THX-B (4mg/Kg) were administrated intraperitoneally twice a week. A) Tumor volume was measured using a caliper. Analysis of (A) primary tumor growth and (B) distal metastasis after treatment. Distal metastases were analyzed measuring luciferase expression in the lungs using IVIS system. Error bars represent s.e.m of 3 experiment (n = 12 mice, vehicle group, n = 12, antiPD-L1 group; n=8 antiPDL1 and THX-B group). p values were calculated by two-tailed Student's t test.

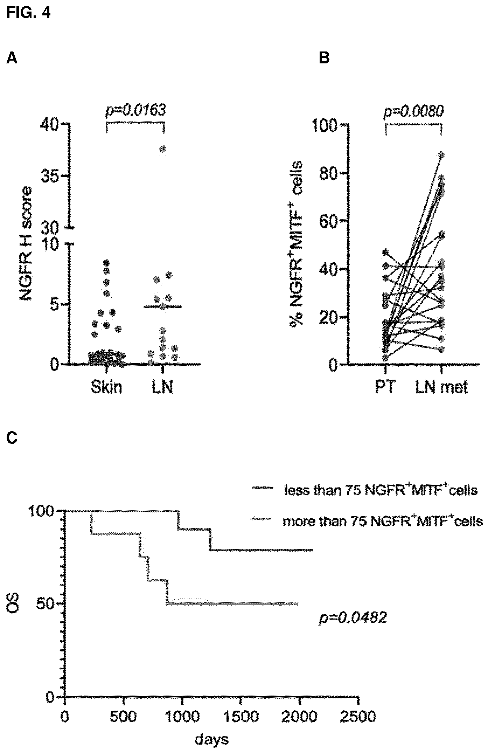

**Figure 4****. Analysis of p75^{NTR}/NGFR in human samples.** a) Histological H score for p75^{NTR}/NGFR staining in skin and LN sections of a cohort of 44 melanoma patients (n = 26 skin/soft tissue samples and n = 18 LN samples). p value was calculated using Mann-Whitney test. **b),** Analysis of the percentage of p75^{NTR}/NGFR ⁺MITF⁺ tumor cells in matched skin and LN samples of patients in Stage III/IV melanoma (n=21 pairs of samples). *p* value was calculated using paired Wilcoxon matched-pairs signed rank test. **c)** Kaplan-Meier survival curve representing overall survival (OS) of stage II/III melanoma patients according to the quantification of double positive p75^{NTR}/NGFR ⁺MITF⁺ cells in LN biopsies (n = 13 samples with less than 75 NGFR⁺MITF⁺ cells and n = 12 with more than 75 p75^{NTR}/NGFR ⁺MITF⁺ cells). *p* value was calculated by Log-rank test.

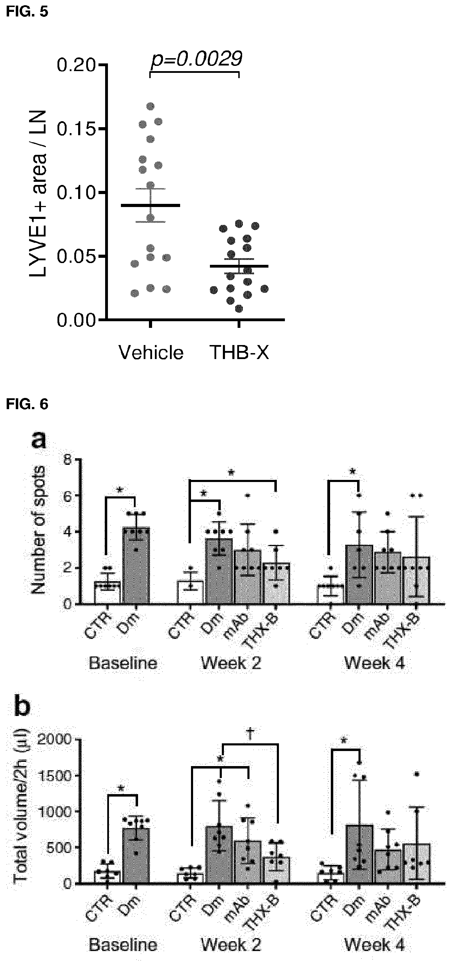

**Figure 5****. p75^{NTR}/NGFR inhibition by THX-B reduces lymphangiogenesis.** Analysis of lymphatic area in histological sections of inguinal LNs of 8-10 weeks-old males with flank tumors originated by injected B16F1-R2 cells (200.000 cells/flank). Animals were treated intra-peritoneally with 2.5 mg/Kg of THX-B twice a week, starting on day 7 post-injection of tumor cells. Animals were sacrificed at 21 days. LN sections were stained with an antibody against the lymphatic marker LYVE-1. Quantification of LYVE+ area was performed with ImageJ software and was normalized to total LN area in each section. Error bars represent s.e.m of 1 experiment (n = 6 LNs, 3 whole sections analyzed per nodule). *p* values were calculated by two-tailed Student's t test.

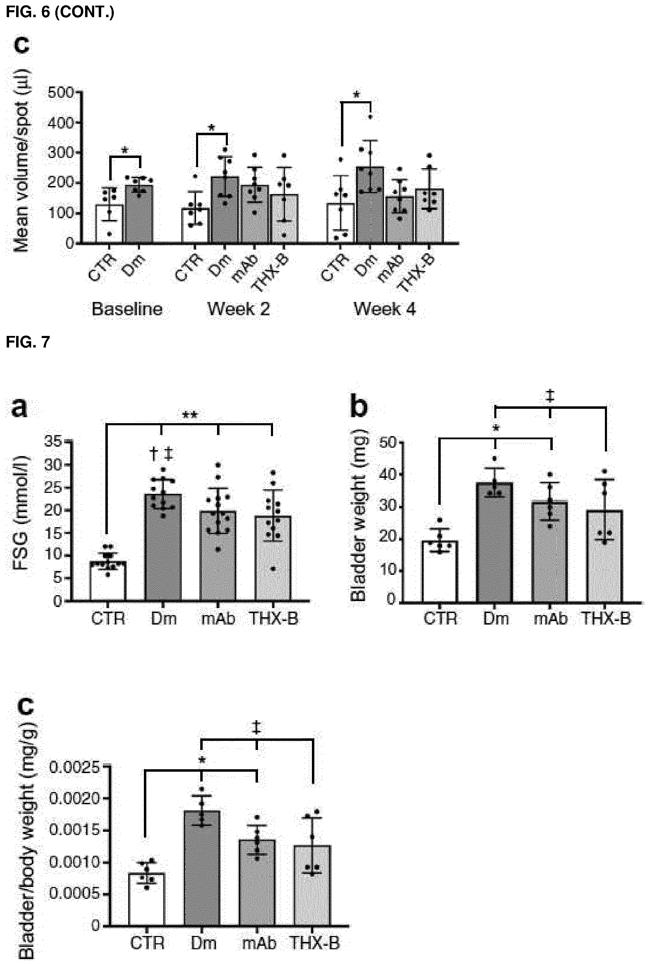

**Figure 6****. Voiding spot assay over a 2 h period.** (a) Number of spots, reflecting voiding frequency; **(b)** Total voided volume after 2 h; **(c)** Mean volume per spot; ; *n=* 4-8 mice in each group at each time point; **p*<0.05 vs control, ^{†}*p*<0.05 vs THX-B. All comparisons were done at a particular time point, for Baseline independent t-test, for all other time points one-way ANOVA with post-hoc Tukey test. Data represent mean (SD)
**Figure 7****. Fasting serum glucose (FSG) and bladder weight and morphology after the drug washout period** (3 weeks after the last injection, 8 weeks of diabetes). **(a)** FSG in the diabetic treated mice was significantly lower than in the Dm group despite being significantly higher than control **(b)** Bladder weight shows persistent absence of bladder hypertrophy in the THX-B group compared with Dm and mAb after the washout period. **(c)** Bladder/body weight ratio showed similar pattern to the bladder weight changes. ^{*}*p*<0.05 vs control; ^{†}*p*<0.05 mAb; ^{‡}*p*<0.05 vs THX-B; one-way ANOVA with post-hoc Tukey test. Data represent mean (SD).

### EXAMPLES

The following examples illustrate the present invention:

### THX-B reduces metastasis

Our data show that inhibition by intraperitoneal administration of THX-B impaired both local (lymph node) and distal (lung) spontaneous metastasis in mouse melanoma xenograft models (Figure 1), demonstrating that p75^{NTR}/NGFR inhibition reduces specifically metastasis.

Moreover, we have validated the use of THX-B in pre-clinical models of melanoma using human melanoma cell lines. Our data also showed that THX-B treatment reduce lung spontaneous metastasis in immunodeficient models (Figure 2).

We analyzed the effect of THX-B in combination with immunotherapy (anti-PD-L1) in melanoma. Analysis of the combination of THX-B with anti-PD-L1 showed that while anti-PD-L1 treatment decreased tumor growth of B16-F10 cells, it failed to block melanoma metastasis (Figure. 3), however the combination of anti-PD-L1 with THX-B inhibited both tumor and metastasis (Figure. 3).

### p75^{NTR} expression in metastatic cells in sentinel lymph nodes correlates with disease outcome

In order to define the use of p75^{NTR}/NGFR in early metastatic disease, we analyzed the presence of p75^{NTR}/NGFR in circulating small extracellular vesicles (sEVs) and lymph node metastasis. Histological analysis of p75^{NTR}/NGFR expression in a cohort of 44 Stage III/IV melanoma patients showed that p75^{NTR}/NGFR expression was higher in metastatic LN tissues than in skin lesions (Figure. 4a). Moreover, evaluation of another independent cohort of 25 Stage II/III melanoma patients with matched primary tumor and LN tissue biopsies uncovered an increase in the frequency of p75^{NTR}/NGFR ⁺ tumor cells (identified by co-staining with the melanoma marker MITF) in LN metastases compared to matched primary tumors (Figure. 4b). Remarkably, the number of p75^{NTR}/NGFR ⁺MITF⁺ cells in LNs biopsies predicted patient survival (Figure. 4c).

We have analyzed the presence of p75^{NTR}/NGFR in extracellular vesicles from plasma and lymphatic drainage obtained from melanoma patients (defined as seroma). We found that p75^{NTR}/NGFR can be detected in extracellular vesicles from seroma samples from Stage III melanoma patients. further methods (e.g. ELISA) can be used to detect p75^{NTR}/NGFR in secreted extracellular vesicles as prognostic marker in melanoma and other diseases with high p75^{NTR}/NGFR.

Consistent with this fact, the treatment with THX-B decreased lymph node lymphangiogenesis denoted by a reduction of Lyve-1+ staining in inguinal lymph nodes of animals with flank tumors originated by F1-R2 cells (Figure 5) which support that THX-B reduces tumor p75^{NTR}/NGFR-driven lymphangiogenesis.

We have tested the administration and stability of THX-B in mouse models by intra peritoneal and oral administration in melanoma. For ophthalmic indications THX-B delivery of the drug via non-invasive subconjunctival route. Topical delivery is also a viable option for inflammatory diseases (e.g. psoriasis) or skin melanoma.

We have analyzed the pharmacodynamics of THX-B in males and female mice by intraperitoneal injection observing that detection levels dropped 1 hour after injection (4mg/Kg), suggesting a short half-life of the molecule. Specific analysis of organ distribution in females showed that compound can be detected in lymph nodes after 20 hours of the injection (4mg/Kg), however in other organs (kidney, lung, liver, blood) was not detected. Finally, we evaluated other routes of administration such as oral gavage and we observed that half-life was similar at 10mg/Kg dose but sustained over time at 50mg/Kg. Regarding tissue distribution after oral dose 50mg/Kg, in all the tissues except the lymph nodes the compound is detected even at 24h showing suggesting a homogeneous distribution between tissues in concentration ranging from 12 to 20 ng/ml. In lymph nodes compound was detected at 8 hours with a concentration of 9 ng/ml.

### Voiding of bladder is improved with THX-B and proNGF blocker mAb

The micturition behaviour of Dm assessed by the VSA showed a statistically significant increase in voiding frequency, total voided volume and mean volume per micturition compared with control mice at all time points (Fig. 6a-c). Both treatments (proNGF mAb and THX-B) had minimal effects on these parameters, except for a 50% lower total voided volume in the THX-B-treated mice after 2 weeks of treatment (372±73µl vs 802.5±123µl, p<0.05) compared with Dm (Fig. 6a-c).

**Voiding spot assay:** For the voiding spot assay (VSA), mice were placed individually with access to food without water for 2 h. The cage was fitted with a chromatography paper, grade 3 mm CHR (Whatman, GE Healthcare, UK) covered by a fine metallic mesh (2-3 mm pore size). The urine spot pattern was assessed by imaging the chromatography paper with ultraviolet light using SynGene Bioimaging system (USA) connected to GeneSnap software (Version 7.02, USA) and analysed by ImageJ software (Version: 2.0.0-rc-66/1.52n, NIH, USA) using a calibration curve and Void Whizzard free software plugin for FIJI (distribution of ImageJ, Version: 2.0.0-rc-66/1.52n, NIH, USA). Mice were acquainted to the procedure once to get accustomed, then the experiment was done once to obtain the voiding data.

Table 1 shows that the morphology of the bladder is improved following THX-B treatment and that the bladder structure is normal in spite of continuous hyperglycemia (and diabetes).

| **Table 1 Summary of diabetic voiding dysfunction (DVD) changes in our animal model and the observed effects of proNGF/p75^{NTR} axis blockage, compared with Dm** | | | |
|---|---|---|---|
| **Outcome** | **Dm** | **proNGF mAb** | **THX-B** |
| **Bladder weight** | ↑ | ↓ (week 2) | ↓ (week 2,4) |
| | | ⇔ (week 4) | |

| **Morphology** | | | |
|---|---|---|---|
| **Overall thickness** | ↑ | ⇔ | ↓ |
| **Collagen content** | ↑ | ↓ | ↓ |
| **Mucosa** | ↑ | ↓ | ↓ |
| **Detrusor** | ↑ | ↑ | ↓ |

| **VSA** | | | |
|---|---|---|---|
| **Number of spots** | ↑ | ⇔ | ↓ (week 2) |
| **Total volume** | ↑ | ⇔ | ↓ (week 2) |
| **Volume per spot** | ↑ | ⇔ | ⇔ |

| **Cystometry** | | | |
|---|---|---|---|
| **Spontaneous activity** | ↑ | ⇔ | ⇔ |
| **ICI** | ↑ | ⇔ | ↓ (week 4) |
| **Bladder capacity** | ↑ | ↓ (week 2) | ↓ (week 4) |
| **Bladder compliance** | ↑ | ↓ (week 2) | ⇔ |

| **Contractility** | | | |
|---|---|---|---|
| **KCI** | ↓ | ↑ (week 2) | ↑ (week 2,4) |
| **Carbachol** | ↓ | ↑ (week 2) | ↑ (week 2,4) |
| **EFS** | ↓ | ↑ (week 2) | ↑ (week 2,4) |

| **Molecular changes** | | | |
|---|---|---|---|
| **proNGF/NGF** | ↑↑ | ↓ | ↓ |
| **TNF-a** | ↑↑ (week 2,4) | ↓↓ | ↓↓ |

| | | | |
|---|---|---|---|
| ICI, inter-contraction interval VSA, Voiding spot assay EFS, Electric field stimulation | | | |

### Prolonged treatment decreases bladder hypertrophy, and the effect is sustained after drug washout

The Fasting serum glucose (FSG) in both treatment groups was significantly lower than those in the Diabetic mouse/mice (untreated) (Dm) after a 3 week washout period (p < 0.05). Bladder weight in THX-B group maintained a significant difference when compared with Dm (p < 0.05) while mean bladder weight of proNGF mAb-treated mice was not statistically different from Dm despite maintaining similar pre-drug washout bladder weight. (Fig. 7 b) (ESM Table 1) In addition, photomicrographs of the bladders showed that hypertrophy was absent in both treatment groups, suggesting a prolonged effect on blood glucose and bladder morphology. In both the proNGF mAb and the THX-B treatments, the prolonged anatomical physiological effects on the bladder are likely to be due to a prolonged therapeutic effect rather than to the drugs remaining in circulation at therapeutic levels after a 3 week period, given that we have observed that anti-proNGF mAb is detectable in systemic circulation only for 2 weeks in previous studies.

**Animal housing and treatments** All experiments received the ethical approval from the Lady Davis Institute Ethics Board (No. 7859), Montreal, Canada. Housing and handling conformed with the Canadian Council for Animal Care (CCAC). One hundred and forty four 9-week-old C57BL/6 female mice were kept on a 12 h light/dark cycle with free access to food (standard Purina chow, Teklad Global, Wl, USA) and water. Diabetes was induced by i.p. injection of streptozotocin (60 mg/kg in citrate buffer, 10 mmol/l, pH 4.5 on 5 consecutive days) after 4-5 h fasting. Fasting serum glucose (FSG) was checked after 5-7 days using a glucometer (Contour next EZ, Bayer, ON, Canada) from the tail. FSG above 14 mmol/l was considered diabetic. Mice were divided into four groups without pre-considering their weight changes or glucose levels (for diabetic groups): control (42 mice); untreated diabetic (hereafter referred to as Dm; 42 mice); anti-proNGF mAb-treated diabetic mice (30 mice); and small molecule p75^{NTR} antagonist (THX-B)-treated diabetic mice (30 mice). The anti-proNGF mAb has a molecular mass of 150 kDa and inhibits binding of the ligand to p75^{NTR} but does not inhibit binding of the ligand to other TrkA receptor. This inhibitor antagonises ligand-dependent p75^{NTR} functions and, different from other anti-NGF mAbs, it is free of the off-target side effects resulting from inhibiting TrkA. We have observed that the molecule is detectable in systemic circulation for 2 weeks. The THX-B small molecule ((1,3-diisopropyl-1-[2-(1,3-dimethyl-2,6-dioxo-1,2,3,6-tetrahydro-purin-7-yl)-acetyl]-urea); molecular mass 540 Da) is a synthetic chemical with an estimated systemic circulation half-life in vivo of 6-8 h. It inhibits proNGF binding to p75^{NTR} and also inhibits ligand-independent signals by p75^{NTR}.

Without blinding, i.p. injections of the drug molecules or the vehicle (PBS) were given weekly for four weeks, starting after 2 weeks of diabetes confirmation. A single starting proNGF mAb i.p. injection of 100 µg/mouse in 150 µl PBS was followed by 60 µg/mouse in 100µl PBS for the following three injections. THX-B was injected i.p. at a weekly dose of 50 µg/mouse in 125 µl PBS. Experimental time points (to carry out cystometry, contractility, protein extraction and histology) were baseline (two weeks after diabetes diagnosis included control and Dm groups only), week 2 after treatment (following 2 injection cycles) and week 4 after treatment (following 4 injection cycles). Body weight was measured weekly and FSG fortnightly for all groups. Six mice from each group were kept for another 2 weeks after the end of the study duration to observe the washout/prolonged effect of the molecules.

## Claims

**1.** A composition comprising THX-B and at least one pharmaceutically acceptable excipient for use in the treatment or prevention of cancer and/or metastasis.

**2.** The composition for use according to the preceding claim, wherein THX-B is administered intravenously, intraperitoneally, orally, subcutaneously, transdermally, subconjunctivally, topically or intravitreously.

**3.** The composition for use according to any one of the preceding claims, wherein THX-B is administered at a dose of between 0.1 and 100 mg/kg, preferably between 1 and 60 mg/kg, more preferably between 3 and 50 mg/kg.

**4.** The composition for use according to any one of the preceding claims, wherein the cancer is a p75^{NTR} expressing cancer.

**5.** The composition for use according to the preceding claim, wherein the cancer is selected from pancreatic cancer, basal cell carcinoma, cervical squamous cell carcinoma (SCC), oral SCC, head and neck SCC, lung SCC, glioma, glioblastoma, esophageal SCC, medulloblastoma, breast cancer, thyroid cancer, ovarian cancer, renal cell carcinoma, malignant peripheral nerve tumors, schwannomas and melanoma or wherein the metastasis is from any one of pancreatic cancer, basal cell carcinoma, cervical squamous cell carcinoma (SCC), oral SCC, head and neck SCC, lung SCC, glioma, esophageal SCC, medulloblastoma, breast cancer, thyroid cancer, ovarian cancer, renal cell carcinoma, malignant peripheral nerve tumors, schwannomas and melanoma.

**5.** The composition for use according to any one of the two preceding claims, wherein the cancer is melanoma or wherein the metastasis is from melanoma.

**6.** The composition for use according to the preceding claim, wherein the melanoma is skin melanoma.

**7.** The composition for use according to any one of the preceding claims, wherein THX-B is administered in combination with immunotherapy.

**8.** The composition for use according to the preceding claim, wherein the immunotherapy is selected from inhibitors against CTLA-4, PD-1, PD-L1, Lag-3, Tim-3, TIGIT or a combination thereof, preferably inhibits CTLA-4, PD-1 or PD-L1, more preferably inhibits PD-L1.

**9.** An early metastasis prognostic method comprising determining the expression levels of p75^{NTR} in a sample from a subject afflicted with cancer; wherein the sample comprises or consists essentially in tumor cells from the sentinel lymph node, circulating extracellular vesicles, circulating soluble p75^{NTR} or fragments thereof or circulating tumor cells; and wherein higher expression levels of p75^{NTR} in said sample are indicative of a worse prognosis.

**10.** The early metastasis prognostic method according to the preceding claim, wherein the cancer is melanoma.

**11.** The early metastasis prognostic method according to any one of the two preceding claims, wherein the subject is human.

**12.** The early metastasis prognostic method according to any one of the two preceding claims, wherein the p75^{NTR} expression levels are analysed by immunohistochemistry, qPCR, flow cytometry or quantitative ELISA.

**13.** The composition comprising THX-B and at least one pharmaceutically acceptable excipient for use according to any one of claims 1 to 8, for use in the treatment or prevention of cancer and/or metastasis in a subject afflicted with cancer who has a worse prognosis in the early metastasis prognostic method according to any one of claims 9 to 12.

**14.** A composition comprising THX-B and at least one pharmaceutically acceptable excipient for use in the prevention of lymphangiogenesis.

**15.** The composition for use according to the preceding claim, for the prevention of lymphatic disorders dependent on p75^{NTR}.
